# EUROPÄISCHE PATENTSCHRIFT

(11) **EP 2 532 641 B1**
(45) Veröffentlichungstag und Bekanntmachung des Hinweises auf die Patenterteilung: **19.08.2015**
(21) Anmeldenummer: 11004625.7
(22) Anmeldetag: 07.06.2011
(51) Int. Cl.: C07C 17/358, C07C 25/13, C07C 25/18

(54) **Verfahren zur Isomerisierung**
Isomerisation method
Procédé d'isomérisation

(43) Veröffentlichungstag der Anmeldung: 12.12.2012
(73) Patentinhaber: Merck Patent GmbH, 64293 Darmstadt (DE)
(72) Erfinder: Maillard, David, Dr., 64285 Darmstadt (DE); Kriegbaum, Markus, 64319 Pfungstadt/Hahn (DE); Wächtler, Andreas, Dr., 64295 Darmstadt (DE); Krattinger, Philipp, Dr., 64291 Darmstadt (DE); Schäfer, Ralf, 63225 Langen (DE)

(56) Entgegenhaltungen:
- DE-A1-102009 058 574
- DATABASE WPI Week 199725 Thomson Scientific, London, GB; AN 1997-276717 XP000002659224, & JP 9 100286 A (SHINETSU CHEM IND CO LTD) 15. April 1997 (1997-04-15)
- DATABASE WPI Week 200463 Thomson Scientific, London, GB; AN 2004-646561 XP000002659225, & JP 2004 256490 A (NAGASE CIBA KK) 16. September 2004 (2004-09-16)

## Beschreibung

Die vorliegende Erfindung betrifft ein Verfahren zur Herstellung von trans-1,4-disubstituierten Cyclohexanverbindungen mit mindestens einem von Benzol abgeleiteten Rest in 1- oder 4-Stellung, das einen Isomerisierungsschritt umfasst, der einen oder mehrere cis-konfigurierte Cyclohenxanringe unter Verwendung einer starken Säure und in Gegenwart einer zusätzlichen Carbokationen bildenden Verbindung in die trans-Konfiguration überführt.

Funktionale Chemikalien werden nicht nur durch ihre vorhandenen funktionellen Gruppen, sondern auch maßgeblich durch ihre Stereochemie, also ihrem räumlichen Aufbau, beeinflusst. Ein nicht unwesentlicher Teilbereich der Stereochemie ist einfachen nicht-aromatischen Ringen und der relativen Lage von Substituenten an diesen Ringen gewidmet.

Ein Grundproblem für die Stereochemie stellt die cis-trans-Isomerie von Substituenten an Cyclohexanen dar. Im Folgenden wird speziell auf die Isomerie von 1,4-substituierten Cyclohexanderivaten eingegangen.

Eine bekannte Reaktion ist die cis-trans-Isomerisierung von 4-Alkylcyclohexylbenzolen. Dabei lässt sich der phenylsubstituierte Cyclohexanring durch geeignete Maßnahmen dahingehend isomerisieren, dass aus einem cis-trans-Gemisch überwiegend das 1,4-trans-Isomer erhalten wird (JP 2004-256490 A; JP 9 100286 A bzw. Derwent WPI 2004-646561/AN). Im Gegensatz zur vorliegenden Erfindung findet die Isomerisierung an einem elektronenreichen Aromaten statt.

Für Isomerisierungsverfahren werden bislang starke Basen wie Kalium-tert-butylat oder Fluoridionen (DE102005034067 A1) eingesetzt. Diese Isomerisierungsverfahren sind jedoch in vielen Fällen mit nicht zufriedenstellenden Ausbeuten verbunden und/oder ergeben aufgrund von unerwünschten Nebenreaktionen ein seinerseits aufwendig zu trennendes Produktgemisch. Produktgemische treten insbesondere bei fluorierten Aromaten deswegen auf, weil unter basischen Bedingungen durch Arinbildung und in deren Folge Poly- oder Oligomerisation geschehen kann oder weil die Base als Nucleophil die Fluorposition unter Substitution angreift.

Die Chemie von Carbokationen unter dem Einfluss von starken Säuren (Supersäuren) wurde wiederholt zusammengefasst, zuletzt z. B. von George A. Olah in "Superacid Chemistry", 2. Ed., Wiley, 2009. Die Anwendung dieser Chemie auf die cis/trans-Stereochemie von 1,4-substituierten Cyclohexanen wurde bisher nicht untersucht.

Es wurde nun ein allgemein anwendbares Verfahren zur Herstellung von trans-substituierten Cyclohexanverbindungen der Formel I gefunden, 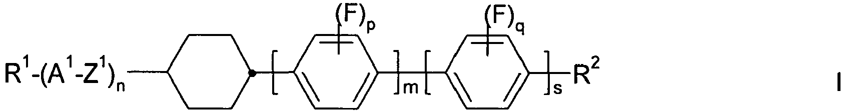 worin
- R¹: einen unsubstituierten, geradkettigen Alkylrest mit bis zu 9 C-Atomen oder -CH(CH₃)CH₃,
- R²: H, Halogen, bevorzugt F, einen Alkylrest mit bis zu 9 C-Atomen, einen Perfluoralkylrest mit bis zu 9 C-Atomen oder einen Perfluoralkoxyrest mit bis zu 9 C-Atomen, für s > 0 auch Alkoxy mit bis zu 9 C-Atomen,
- n: 0, 1 oder 2,
- m: 1 oder 2,
- s: 0, 1 oder 2,
- p: 1, 2, 3 oder 4,
- q: 0, 1, 2, 3 oder 4,
- A¹: trans-1,4-Cyclohexylen, und
- Z¹: -(CH₂)ₜ- mit t = 0, 1, 2, 3 oder 4, bevorzugt t = 0, 2 oder 4,
bedeuten,
das die Umsetzung von Verbindungen der Formel I entsprechenden 1,4-cis-Cyclohexanverbindungen in Gegenwart einer starken Säure, bevorzugt einer Supersäure, und
in Gegenwart einer zusätzlichen Carbokationen bildenden Verbindung umfasst, wobei die Cationen bildende Verbindung eine oder mehrere Zusätze in Form eines tertiären oder sekundären Alkylhalogenids oder -sulfonats, eines Neopentylhalogenids oder eines tertiären oder sekundären Alkohol bedeutet.

Als Edukte werden typischerweise Verbindungen der Formel II 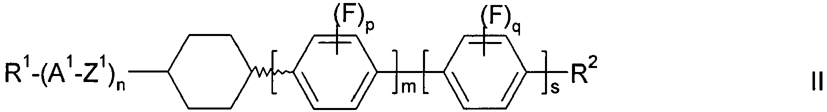 eingesetzt, die eine cis-Konfiguration am Cyclohexanring aufweisen, oder Gemische, die einen Anteil mit der cis-Konfiguration enthalten, wobei R¹, R², Z¹, m, n, p, q und s wie für Formel I definiert sind.

Die Carbokationen-bildende Verbindung wird im Folgenden als Katalysator bezeichnet. Das Carbokation bildet sich durch Reaktion des Katalysators mit der Säure. Der Katalysator wird bevorzugt in substöchiometrischen Mengen eingesetzt, d.h. in der Praxis werden bevorzugt Mengen von 0,001 bis 95 mol% bezogen auf das zu isomerisierende Substrat eingesetzt. Besonders bevorzugt ist eine Menge an zugesetztem Katalysator von 0,01 bis 10 mol%. Abhängig von der Herstellung des Ausgangsmaterials ist in manchen Fällen eine Zugabe des Katalysators nicht nötig, weil bereits genügend Verunreinigungen enthalten sind, die die Rolle des Katalysators übernehmen. Bevorzugt wird der Katalysator jedoch eigens zugegeben.

Das erfindungsgemäße Verfahren zeichnet sich durch eine schonende Verfahrensweise aus, durch einen äußerst hohen trans-Anteil im Isomerisierungsprodukt und durch wenig Nebenprodukte. Die Abtrennung des zugesetzten Katalysators ist unproblematisch.

Als starke Säuren eignen sich in erster Linie solche mit einem H₀-Wert kleiner -11 (Supersäuren), bevorzugt perfluorierte Alkylsulfonsäuren, insbesondere Trifluormethansulfonsäure, Nonafluorbutansulfonsäure und deren Derivate, wie Tris(trifluorsulfonyl)methan oder (CF₃SO₂)₂NH als Beispiele. Wo nötig, kann die Stärke der Säure durch Kombination mit einer Lewis-Säure noch verstärkt werden. Dies kann besonders dann vorteilhaft sein, wenn man Alkene als Katalysatoren einsetzt. Viele weitere superacide Systeme in der flüssigen Phase sind aus der einschlägigen Literatur bekannt (vgl. Kapitel 2 in Olah, Superacid Chemistry, oben zitiert), wie Fluoroschwefelsäure und Gemische mit Lewissäuren wie SbF₅ etc ('magic acid'). Besonders bevorzugt sind allgemein nicht oxidierende Systeme, also solche die keine Schwefelsäure, Perschwefelsäure oder Schwefeltrioxid enthalten. Die starke Säure hat carbokationen-stabilisierende oder superelektrophile Eigenschaften und ermöglicht die kationenvermittelte Reaktion. Starke Säuren im Sinne der Erfindung sind daher solche Säuren, die geeignet sind, mit der Carbokationen bildenden Verbindung Carbokationen zu generieren.

Das Verfahren wird in Gegenwart eines sekundären oder tertiären Alkylhalogenids oder -sulfonats oder eines sekundären oder tertiären Alkohols als Katalysator durchgeführt. Bevorzugt wird als Katalysator ein tertiäres Halogenid, ein Halogenid an einem Brückenkopf eines aliphatischen Kohlenwasserstoffpolyzyklusses oder ein Neopentylhalogenid eingesetzt. Als Halogenide sind in diesem Zusammenhang Chlor, Brom oder Iod, insbesondere Chlorid bevorzugt. Anstelle eines Halogenids ist jeweils auch eine Hydroxylgruppe möglich. Bei den Katalysatoren kann es sich auch um geminal mehrfach halogenierte Verbindungen, wie beispielsweise Haloform oder Tetrahalomethan, handeln. Beispiele von guten Carbokationenbildnern sind brückenkopfsubstituierte Adamantanverbindungen (bevorzugt Alkohol, Halogenid oder Alkylether) wie z.B. 1-Adamantanol oder 1-Halogenadamantan, außerdem Norborneol und Norbornylchlorid. Besonders wirtschaftlich aufgrund der guten Verfügbarkeit und Wirksamkeit ist die Verwendung von tert-Butylchlorid (2-Chlor-2-methylpropan) als Katalysator.

Der Katalysator wird bevorzugt in katalytischen Mengen eingesetzt, insbesondere in einer Menge, die noch geringer ist als die molare Menge der zugesetzten starken Säure. Bevorzugte Mengen des Katalysators sind 0,01 bis 10 mol% bezogen auf das Produkt oder 1 bis 90 %, bevorzugt 5 bis 20 % bezogen auf die Säure.

Die Zugabe von größeren als den angegebenen oder gar stöchiometrischen Mengen des Katalysators kann ggf. von Nachteil für das Verfahren sein.

In der Praxis sind in den Rohstoffen für das Verfahren bisweilen bereits Verunreinigungen enthalten, die einem der genannten Katalysatorzusätze entsprechen. In diesen Fällen erübrigt sich unter Umständen ein solcher Zusatz, weil das Ausgangsmaterial, insbesondere wenn es über eine Addition eines Metallaryls an ein Cyclohexanon mit anschließender Wassereliminierung und Hydrierung gewonnen worden ist, bereits katalytisch wirksame Spezies als Verunreinigung enthält.

Das Verfahren wird bevorzugt in einem chlorierten oder fluorierten Lösungsmittel, wie z. B. in Dichlormethan, 1,3-Difluorbenzol, Trifluormethylbenzol, 1,2,3,4- oder 1,2,3,5-Tetrafluorbenzol, Pentafluorbenzol oder chlorierten Fluorkohlenwasserstoffen durchgeführt. Geeignete Lösungsmittel sind aus der Chemie in Supersäuren bekannt (vgl. G. A. Olah, div. Publikationen).

Die Reaktionstemperatur im Verfahren liegt bevorzugt kleiner 0 °C, bevorzugt kleiner -20 °C. Sie liegt bevorzugt zwischen 30 und -180 °C, besonders bevorzugt zwischen 0 °C und -100 °C und ganz besonders bevorzugt zwischen -30 °C bis -78 °C. Durch die niedrige Verfahrenstemperatur werden wenig Nebenprodukte erzeugt. Auch empfindliche Edukte können eingesetzt werden.

Das gewünschte Produkt wird trotzt der niedrigen Reaktionstemperatur in der Regel nach einer Reaktionszeit von 0,1 h bis 4 h erhalten.

Die im erfindungsgemäßen Verfahren eingesetzten Cyclohexane mit einem zu isomerisierenden cis-Anteil werden nach üblichen Methoden hergestellt. Bewährte Ausgangsverbindungen sind 4-substituierte Cyclohexanone, z. B. 4-Alkyl-cyclohexanone, 4-(4-Alkyl-cyclohexyl)-cyclohexanon, Cyclohexan-1,4-dion oder die entsprechenden Ketale. Aus diesen Edukten bieten sich ein bewährter Synthesewege zu den Cyclohexanen an: Durch Addition von Grignard- oder lithiierten Verbindungen an die Carbonylgruppe mit anschließender Eliminierung und Hydrierung der Cyclohexene an gängigen Katalysatoren. Aus den jeweiligen Hydrierungen resultieren in der Regel cis/trans-Gemische mit einem trans-Gehalt kleiner 85 %, meistens wesentlich darunter. Ein bevorzugtes Verfahren ist daher dadurch gekennzeichnet, dass die Konfiguration des Reaktionsprodukts an jedem 1,4-substituierten Cyclohexanring zu 95 % oder mehr der trans-Konfiguration entspricht, wobei vorzugsweise die Konfiguration des Edukts an mindestens einem 1,4-substituierten Cyclohexanring zu 90 % oder weniger, bevorzugt zu 85 % oder weniger, der trans-Konfiguration entspricht. Das Produkt besitzt vorzugsweise zu 97 % oder mehr die all-trans-Konfiguration an den 1,4-Cyclohexanresten.

Ein bevorzugtes Verfahren gemäß der Erfindung ist außerdem dadurch gekennzeichnet, dass es als einen weiteren Verfahrensschritt vor der erfindungsgemäßen Isomerisierung mit der Säure eine Hydrierung an einem Cyclohexenring, einem Benzolring oder einem Cyclohexadienring umfasst, wobei dieser Ring in einen Cyclohexanring überführt wird. Vorzugsweise wird genau dieser Ring bei der Isomerisierung in die trans-konfiguration überführt, soweit er in der cis-Konfiguration aus der Hydrierung hervorgeht.

Das erfindungsgemäße Verfahren stellt eine effiziente Methode zur Isomerisierung des unerwünschten cis-Anteils der 1,4-Cyclohexanderivate dar. Das Verfahren kann zur direkten Erhöhung des trans-Anteils von cis/trans-Gemischen verwendet werden, selbst wenn der Anteil des trans-Isomeren bereits 85 % oder mehr beträgt. Der trans-Anteil nach der Isomerisierung beträgt vorzugsweise 95 % oder mehr, besonders bevorzugt 97 % oder mehr und insbesondere 99% oder mehr. Der Gehalt bezieht sich auf das Rohgemisch. Ebenso kann das Verfahren zur Isomerisierung von Rückständen mit erhöhtem cis-Anteil aus der Anreicherung von trans-Isomeren, z.B. Mutterlaugen aus der Kristallisation, verwendet werden. So können auch nicht verwertbare Reste an ciskonfiguriertem Material durch Isomerisierung wiederverwertet werden.

In einer bevorzugten Ausführungsform der Erfindung sind in der Formel I die Parameter n = 1, m = 0, s = 1 und Z¹ eine Einfachbindung. Bevorzugt ist q = 1 oder 2. R¹ ist bevorzugt ein linearer Alkylrest mit 1 bis 7 C-Atomen oder ein Isopropylrest, R² ist bevorzugt F, ein linearer Alkylrest oder ein linearer Perfluoroalkylrest bzw. Perfluoroalkoxyrest.

Der terminale Benzolring der Verbindung der Formel I ist bevorzugt ein Rest ausgewählt aus den Formeln: 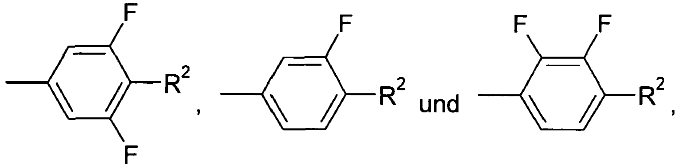 und insbesondere der 3,5-Difluorphenyl oder der 2,3-Difluorphenylrest.

Besonders bevorzugte Produkte der Formel I sind daher 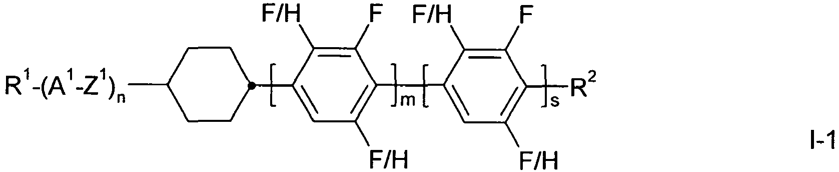 oder 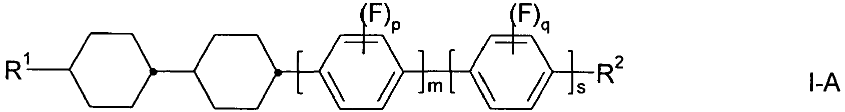 darunter bevorzugt 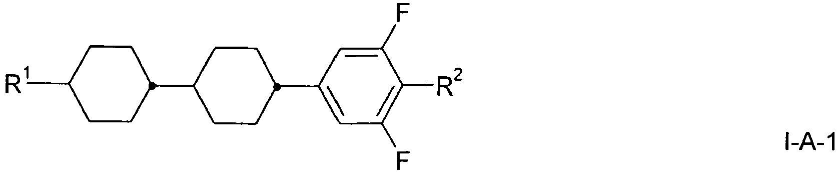 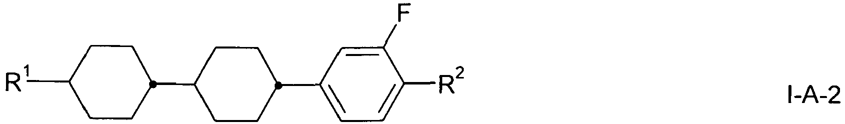 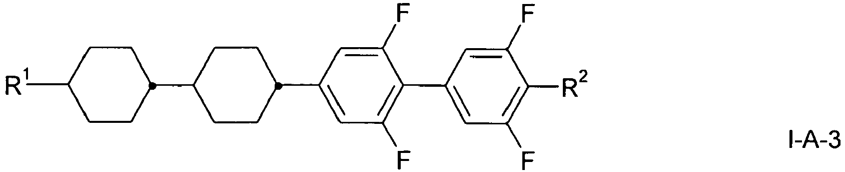 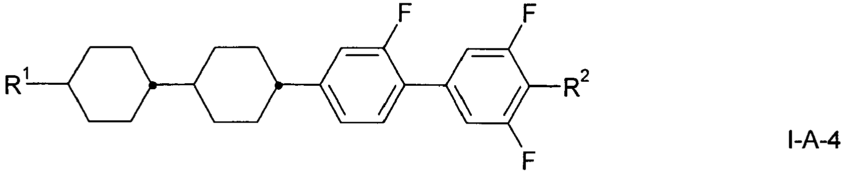 sowie 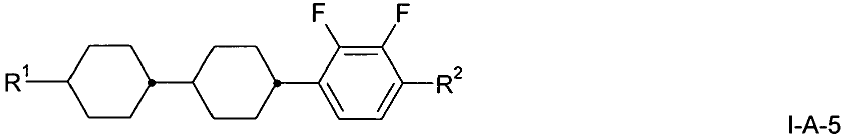 oder 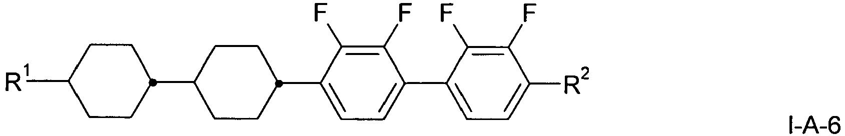 worin R² jeweils bevorzugt H, F, einen Alkylrest, einen Perfluoralkylrest oder einen Perfluoralkoxyrest, besonders bevorzugt F oder einen linearen Alkylrest, darstellt.

Eine besonders hohe Reinheit der all-trans-Verbindungen wird in der Regel schon nach einem einzigen Kristallisationsschritt erreicht, um Nebenprodukte, Katalysatorreste und die geringen cis-Anteile abzutrennen. Ohne eine effektives Isomerisierungsverfahren werden zur Abtrennung der cis-Isomere in der Regel mehrere Kristallisationsstufen benötigt und die Kristallisation des trans-Produkts wird durch das anwesende cis-Isomer erschwert. Die Ausbeute ist deshalb nach dem erfindungsgemäßen Verfahren verbessert.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z.B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

In den Diagrammen bedeuten die Ringe 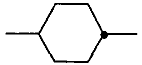 (mit Punkt) einen 1,4-trans-substituierten Cyclohexanring, und 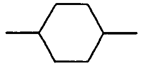 einen 1,4-substituierten Cyclohexanring mit gemischter cis- und trans-Konfiguration oder überwiegend cis-Konfiguration.

Weitere bevorzugte Verfahrensvarianten lassen sich den Beispielen entnehmen, deren Details - auch verallgemeinert nach allgemeiner Fachkenntnis - repräsentativ für bevorzugte Ausführungsformen des erfindungsgemäßen Verfahren und seiner Produkte sind.

### Beispiele

Die Reaktionsprodukte werden mittels HPLC untersucht, um die cis/trans-Anteile und den Anteil an Nebenprodukten zu ermitteln.

### Beispiel 1

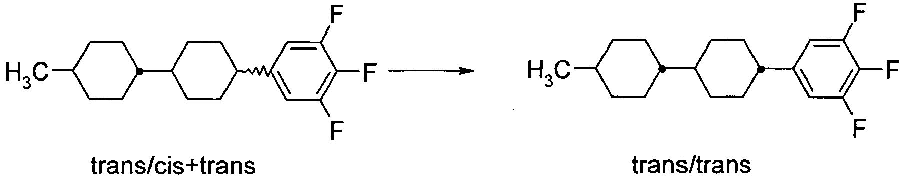

In 90 g wasserfreiem Dichlormethan (zur Analyse) werden 20 g des Edukts (64 mmol, 44 % cis an mittlerem Ring) gelöst und auf -45 °C gekühlt. Zu der Suspension gibt man 2 g Trifluormethansulfonsäure (13 mmol; 1,2 ml). Dann werden 0,15 g 1-Adamantanol (1,0 mmol) in 9 g Dichlormethan zugetropft. Der Ansatz wird bei -40 bis -45 °C 1 bis 3 h weiter gerührt, wobei gelegentlich eine Probe zur Bestimmung des Isomerisierungsgrades entnommen wird. Nach vollständiger Isomerisierung (Probe ist nach 1 bis 2 h vollständig isomerisiert) wird der Ansatz aufgearbeitet.

Zur Aufarbeitung wird der Ansatz mit 38 ml Wasser versetzt und auf ca. 20 °C erwärmt. Die organische Phase wird abgetrennt, mit 22 g triNatriumcitrat-Lösung (25 % in Wasser) und 2 x 20 ml Wasser gewaschen und am Rotationsverdampfer zum Rückstand eingeengt. Der Rückstand (20,2 g) wird mit Heptan aufgenommen und nochmals eingeengt. Das Rohprodukt (leicht gelbliche Kristalle) enthält 0,7 % cis/trans-Anteil, der Anteil an dem gewünschten trans,trans-Produkt beträgt 98,3 % (HPLC). Ein nicht näher bestimmtes weiteres Nebenprodukt mit ähnlicher Retentionszeit ist zu 0,7 % enthalten, außerdem Adamantanderivate.

Die Aufreinigung erfolgt durch Aufnehmen in Heptan und Filtration über 10 g in Heptan angeschlemmtes Kieselgel. Das Filtrat wird am Rotationsverdampfer eingeengt (19,7 g). Der Rückstand wird in heißem Ethanol gelöst und das fast farblose, sehr reine all-trans-Produkt in der Kälte auskristallisiert (16,2 g, Ausbeute 81 %, >99,8 %).

### Beispiel 2

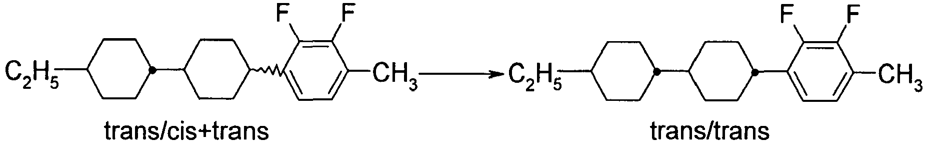

In 650 mL wasserfreiem Dichlormethan (zur Analyse) werden 168 g des Edukts (525 mmol, 59,3 % cis an mittlerem Ring) gelöst und auf -40/-45 °C gekühlt. Zu der Suspension gibt man 9,5 mL Trifluormethansulfonsäure (108 mmol). Dann werden 1,26 g 1-Adamantanol (8,3 mmol) in 50 mL Dichlormethan zugetropft. Der Ansatz wird bei -40 bis -45 °C 1 bis 2 h weiter gerührt, wobei gelegentlich eine Probe zur Bestimmung des Isomerisierungsgrades entnommen wird. Nach vollständiger Isomerisierung (Probe war nach 30min vollständig isomerisiert) wird der Ansatz aufgearbeitet.

Zur Aufarbeitung wird der Ansatz mit einer Mischung aus 175 g Eis und 350 mL Salzsäure 25% versetzt und auf ca. 20 °C erwärmt. Die organische Phase wird abgetrennt, mit 175 mL Natriumhydrogencarbonat-Lösung (5 % in Wasser) und 175 ml Wasser gewaschen und am Rotationsverdampfer zum Rückstand eingeengt. Der Rückstand (168,8 g) wird mit Heptan aufgenommen. Das Rohprodukt (leicht gelbliche Kristalle) enthält 0,23 % cis/trans-Anteil, der Anteil an dem gewünschten trans,trans-Produkt beträgt 99,7 % (GC).

Die Aufreinigung erfolgt durch Aufnehmen in 300 mL Heptan und Filtration über in Heptan angeschlemmtes Kieselgel. Das Filtrat wird am Rotationsverdampfer eingeengt (161,7 g). Der Rückstand wird in heißem Ethanol gelöst und das fast farblose, sehr reine all-trans-Produkt in der Kälte auskristallisiert (145,7 g, Ausbeute 86,7 %, >99,9 %).

Weitere Kombinationen der Ausführungsformen und Varianten der Erfindung ergeben sich aus den Ansprüchen.

## Patentansprüche

1. Verfahren zur Herstellung von 1,4-trans substituierten Cyclohexanverbindungen der Formel I 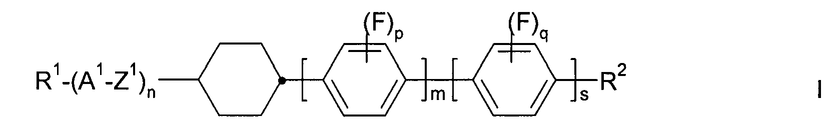 worin
R¹ einen unsubstituierten, geradkettigen Alkylrest mit bis zu 9 C-Atomen oder -CH(CH₃)CH₃,
R² H, Halogen, einen Alkylrest mit bis zu 9 C-Atomen, einen Perfluoralkylrest mit bis zu 9 C-Atomen oder einen Perfluoralkoxyrest mit bis zu 9 C-Atomen, für s > 0 auch Alkoxy mit bis zu 9 C-Atomen,
m 1 oder 2,
n 0, 1 oder 2,
p 1, 2, 3 oder 4,
q 0, 1, 2, 3 oder 4,
s 0, 1 oder 2,
A¹ trans-1,4-Cyclohexylen, und
Z¹ jeweils unabhängig -(CH₂)ₜ- mit t = 0, 1, 2, 3 oder 4,
bedeuten,
das die Umsetzung von Verbindungen der Formel I entsprechenden 1,4-cis-Cyclohexanverbindungen in Gegenwart einer Säure mit einem H₀-Wert kleiner -11 und
in Gegenwart eines zusätzlichen Carbokationen bildenden Katalysators umfasst,
wobei der arbokationen bildende Katalysator einen oder mehrere Zusätze in Form eines tertiären oder sekundären Alkylhalogenids oder -sulfonats, eines Neopentylhalogenids oder eines tertiären oder sekundären Alkohols bedeutet.

2. Verfahren nach Anspruch 1, **dadurch gekennzeichnet, dass** es in Gegenwart einer Perfluoralkylsulfonsäure durchgeführt wird.

3. Verfahren nach Anspruch 1 oder 2, **dadurch gekennzeichnet, dass** die Reaktion in Gegenwart von 1-Adamantanol oder 1-Halogenadamantan durchgeführt wird.

4. Verfahren nach einem oder mehreren der Ansprüche 1 bis 3, **dadurch gekennzeichnet, dass** trans/trans-Bicyclohexan-Verbindungen der Formel IA 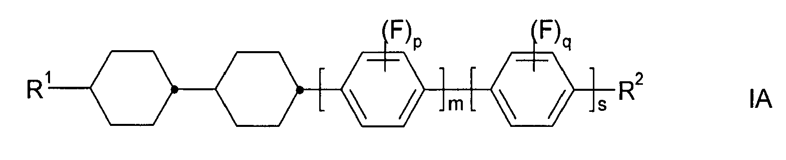 worin m, p, q, s, R¹ und R² wie in Anspruch 1 definiert sind, hergestellt werden.

5. Verfahren nach einem oder mehreren der Ansprüche 1 bis 4, **dadurch gekennzeichnet, dass** die Reaktion bei Temperaturen kleiner oder gleich 0 °C durchgeführt wird.

6. Verfahren nach einem oder mehreren der Ansprüche 1 bis 5, **dadurch gekennzeichnet, dass** die Reaktion in einem chlorierten und/oder fluorierten Kohlenwasserstoff als Lösungsmittel durchgeführt wird.

7. Verfahren nach einem oder mehreren der Ansprüche 1 bis 6, **dadurch gekennzeichnet, dass** es als einen weiteren Verfahrensschritt vor der Isomerisierung hinführend zu den Verbindungen der Formel 1 eine Hydrierung an einem Benzolring, einem Cyclohexenring oder einem Cyclohexadienring umfasst, wobei dieser Ring in einen 1,4-substituierten Cyclohexanring überführt wird.

## Claims

1. Process for the preparation of 1,4-trans-substituted cyclohexane compounds of the formula I 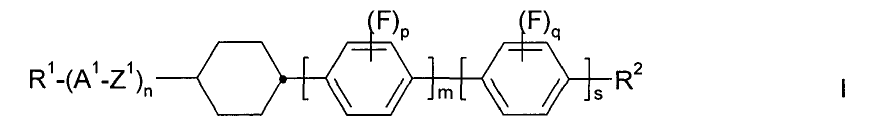 in which
R¹ denotes an unsubstituted, straight-chain alkyl radical having up to 9 C atoms or -CH(CH₃)CH₃,
R² denotes H, halogen, an alkyl radical having up to 9 C atoms, a perfluoroalkyl radical having up to 9 C atoms or a perfluoroalkoxy radical having up to 9 C atoms, for s > 0 also alkoxy having up to 9 C atoms,
m denotes 1 or 2,
n denotes 0, 1 or 2,
p denotes 1, 2, 3 or 4,
q denotes 0, 1, 2, 3 or 4,
s denotes 0, 1 or 2,
A¹ denotes trans-1,4-cyclohexylene, and
Z¹ in each case independently denotes -(CH₂)ₜ-, where t = 0, 1, 2, 3 or 4,
which comprises the reaction of 1,4-cis-cyclohexane compounds corresponding to compounds of the formula I in the presence of an acid having an H₀ value of less than -11, and
in the presence of an additional carbocation-forming catalyst,
where the carbocation-forming catalyst denotes one or more additives in the form of a tertiary or secondary alkyl halide or sulfonate, a neopentyl halide or a tertiary or secondary alcohol.

2. Process according to Claim 1, **characterised in that** it is carried out in the presence of a perfluoroalkylsulfonic acid.

3. Process according to Claim 1 or 2, **characterised in that** the reaction is carried out in the presence of 1-adamantanol or 1-haloadamantane.

4. Process according to one or more of Claims 1 to 3, **characterised in that** trans/trans-bicyclohexane compounds of the formula IA 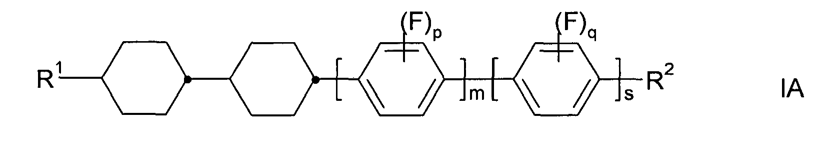 in which m, p, q, s, R¹ and R² are as defined in Claim 1,
are prepared.

5. Process according to one or more of Claims 1 to 4, **characterised in that** the reaction is carried out at temperatures of less than or equal to 0°C.

6. Process according to one or more of Claims 1 to 5, **characterised in that** the reaction is carried out in a chlorinated and/or fluorinated hydrocarbon as solvent.

7. Process according to one or more of Claims 1 to 6, **characterised in that** it comprises, as a further process step before the isomerisation leading to the compounds of the formula I, a hydrogenation on a benzene ring, a cyclohexene ring or a cyclohexadiene ring, where this ring is converted into a 1,4-substituted cyclohexane ring.

## Revendications

1. Procédé de préparation de composés de cyclohexane 1,4-trans-substitué de formule I 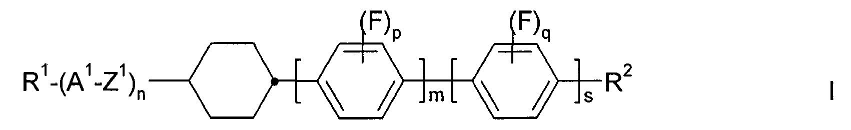 dans laquelle
R¹ désigne un radical alkyle non substitué, à chaîne linéaire, ayant jusqu'à 9 atomes de C ou -CH(CH₃)CH₃,
R² désigne H, halogène, un radical alkyle ayant jusqu'à 9 atomes de C, un radical perfluoroalkyle ayant jusqu'à 9 atomes de C ou un radical perfluoroalcoxy ayant jusqu'à 9 atomes de C, pour s > 0 de même alcoxy ayant jusqu'à 9 atomes de C,
m désigne 1 ou 2,
n désigne 0, 1 ou 2,
p désigne 1, 2, 3 ou 4,
q désigne 0, 1, 2, 3 ou 4,
s désigne 0, 1 ou 2,
A¹ désigne trans-1,4-cyclohexylène, et
Z¹ désigne dans chaque cas indépendamment -(CH₂)ₜ-, où t = 0, 1, 2, 3 ou 4,
comprenant la réaction de composés de 1,4-cis-cyclohexane correspondant aux composés de formule I en présence d'un acide ayant une valeur H₀ inférieure à -11, et
en présence d'un catalyseur supplémentaire de formation de carbocation,
où le catalyseur de formation de carbocation désigne un ou plusieurs additifs sous la forme d'un halogénure ou sulfonate d'alkyle tertiaire ou secondaire, d'un halogénure de néopentyle ou d'un alcool tertiaire ou secondaire.

2. Procédé selon la revendication 1, **caractérisé en ce qu'**il est mis en oeuvre en présence d'un acide perfluoroalkylsulfonique.

3. Procédé selon la revendication 1 ou 2, **caractérisé en ce que** la réaction est effectuée en présence de 1-adamantanol ou de 1-halogéno-adamantane.

4. Procédé selon l'une ou plusieurs parmi les revendications 1 à 3, **caractérisé en ce que** les composés de trans/trans-bicyclohexane de formule IA 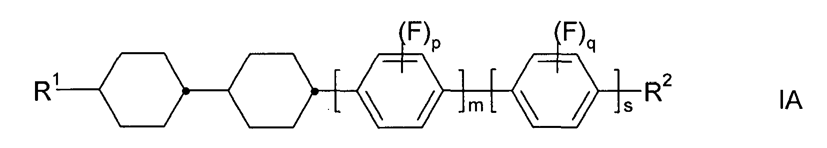 dans laquelle m, p, q, s, R¹ et R² sont tels que définis selon la revendication 1,
sont préparés.

5. Procédé selon l'une ou plusieurs parmi les revendications 1 à 4, **caractérisé en ce que** la réaction est effectuée à des températures inférieures ou égales à 0°C.

6. Procédé selon l'une ou plusieurs parmi les revendications 1 à 5, **caractérisé en ce que** la réaction est effectuée dans un hydrocarbure chloré et/ou fluoré comme solvant.

7. Procédé selon l'une ou plusieurs parmi les revendications 1 à 6, **caractérisé en ce qu'**il comprend, comme étape de procédé supplémentaire avant l'isomérisation conduisant aux composés de formule I, une hydrogénation sur un cycle benzène, un cycle cyclohexène ou un cycle cyclohexadiène, où ce cycle est converti en cycle cyclohexane 1,4-substitué.
